# EUROPEAN PATENT SPECIFICATION

(11) **EP 3 177 316 B1**
(45) Date of publication and mention of the grant of the patent: **08.05.2019**
(21) Application number: 15822240.6
(22) Date of filing: 08.07.2015
(51) Int. Cl.: A61K 38/24, A61P 27/02

(54) **METHOD FOR PREVENTING OR TREATING OCULAR DISORDERS**
VERFAHREN ZUR VERHINDERUNG ODER BEHANDLUNG VON AUGENERKRANKUNGEN
PROCÉDÉ DE PRÉVENTION OU DE TRAITEMENT DE TROUBLES OCULAIRES

(30) Priority: 15.07.2014 US 201462024609 P
(43) Date of publication of application: 14.06.2017
(73) Proprietor: Zietchick Research Institute, LLC, Plymouth, MI 48170 (US)
(72) Inventor: MOVSAS, Tammy, Z., Southfield, MI 48075 (US)
(74) Representative: FRKelly
(86) International application number: PCT/US2015/039542
(87) International publication number: WO 2016/010786

(56) References cited:
- US-A1- 2008 039 372
- US-A1- 2009 136 445
- US-B1- 7 892 753
- THOMPSON D A; OTHMAN M I; LEI Z; LI X; HUANG Z H; EADIE D M; RAO C V: "Localization of receptors for luteinizing hormone/chorionic gonadotropin in neural retina.", LIFE SCIENCES, vol. 63, no. 12, 1998, - 1998, pages 1057-1064, XP002777517,
- DUKIC-STEFANOVIC, SLADJANA ET AL.: 'Chorionic Gonadotropin and Its Receptor Are Both Expressed in Human Retina, Possible Implications in Normal and Pathological Conditions.' PLOS ONE. vol. 7, no. 12, 19 December 2012, pages 1 - 12, XP055385976
- KRINSKY-MCHALE, SHARON J. ET AL.: 'Vision Deficits in Adults with Down Syndrome.' J APPL RES INTELLECT DISABIL. vol. 27, no. 3, May 2014, pages 247 - 263, XP055386013

### SEQUENCE LISTING

<110> Zietchick Research Institute, LLC
<120> METHOD FOR PREVENTING OR TREATING OCULAR DISORDERS
<130> P119510EP00
<140> 15822240.6
   <141> 2015-07-08
<150> US 62/024,609
   <151> 2014-07-15
<160> 2
<170> PatentIn version 3.5
<210> 1
   <211> 92
   <212> PRT
   <213> Homo sapiens
<220>
   <221> CARBOHYD
   <222> (52)..(52)
   <223> N-linked glycosylation
<220>
   <221> CARBOHYD
   <222> (78)..(78)
   <223> N-linked glycosylation
<400> 1
   Ala Pro Asp Val Gln Asp Cys Pro Glu Cys Thr Leu Gln Glu Asp Pro
   1               5                   10                  15
   Phe Phe Ser Gln Pro Gly Ala Pro Ile Leu Gln Cys Met Gly Cys Cys
               20                  25                  30
   Phe Ser Arg Ala Tyr Pro Thr Pro Leu Arg Ser Lys Lys Thr Met Leu
           35                  40                  45
   Val Gln Lys Asn Val Thr Ser Glu Ser Thr Cys Cys Val Ala Lys Ser
       50                  55                  60
   Tyr Asn Arg Val Thr Val Met Gly Gly Phe Lys Val Glu Asn His Thr
   65                  70                  75                  80
   Ala Cys His Cys Ser Thr Cys Tyr Tyr His Lys Ser
                   85                  90
<210> 2
   <211> 145
   <212> PRT
   <213> Homo sapiens
<220>
   <221> CARBOHYD
   <222> (13)..(13)
   <223> N-linked glycosylation
<220>
   <221> CARBOHYD
   <222> (30)..(30)
   <223> N-linked glycosylation
<220>
   <221> CARBOHYD
   <222> (121)..(121)
   <223> O-linked glycosylation
<220>
   <221> CARBOHYD
   <222> (127)..(127)
   <223> O-linked glycosylation
<220>
   <221> CARBOHYD
   <222> (132)..(132)
   <223> O-linked glycosylation
<220>
   <221> CARBOHYD
   <222> (138)..(138)
   <223> O-linked glycosylation
<400> 2
   Ser Lys Glu Pro Leu Arg Pro Arg Cys Arg Pro Ile Asn Ala Thr Leu
   1               5                   10                  15
   Ala Val Glu Lys Glu Gly Cys Pro Val Cys Ile Thr Val Asn Thr Thr
               20                  25                  30
   Ile Cys Ala Gly Tyr Cys Pro Thr Met Thr Arg Val Leu Gln Gly Val
           35                  40                  45
   Leu Pro Ala Leu Pro Gln Val Val Cys Asn Tyr Arg Asp Val Arg Phe
       50                  55                  60
   Glu Ser Ile Arg Leu Pro Gly Cys Pro Arg Gly Val Asn Pro Val Val
   65                  70                  75                  80
   Ser Tyr Ala Val Ala Leu Ser Cys Gln Cys Ala Leu Cys Arg Arg Ser
                   85                  90                  95
   Thr Thr Asp Cys Gly Gly Pro Lys Asp His Pro Leu Thr Cys Asp Asp
               100                 105                 110
   Pro Arg Phe Gln Asp Ser Ser Ser Ser Lys Ala Pro Pro Pro Ser Leu
           115                 120                 125
   Pro Ser Pro Ser Arg Leu Pro Gly Pro Ser Asp Thr Pro Ile Leu Pro
       130                 135                 140
   Gln
   145

## Description

### RELATED APPLICATION

This application claims the benefit of priority of U.S. Provisional Application Serial No. 62/024,609, filed July 15, 2014, entitled **METHOD TO PREVENT PROGRESSION OF OCULAR DISORDERS INVOLVING NEOVASCULARIZATION (SUCH AS DIABETIC RETINOPATHY, AGE-RELATED MACULAR DEGENERATION, RETINOPATHY OF PREMATURITY) UTILIZING HUMAN CHORIONIC GONADOTROPIN ANTAGONISTS**.

### FIELD OF THE INVENTION

The present invention relates to the field of treating and/or preventing ocular disorders,

### BACKGROUND OF THE INVENTION

High levels of vascular endothelial growth factor (VEGF) are known to be associated with the progression of ocular disorders involving retinal and subretinal neovascularization (the growth of pathologic blood vessels); VEGF stimulates endothelial cell growth and neovascularization, and increases retinal vascular permeability. In addition, overexpression of VBGF is known to be associated with tumor progression.

Examples of ocular disorders involving the growth of pathologic blood vessels can be categorized as below. Most of these ocular disorders are VEGF∼associated (i.e, high VEGF levels may contribute to their progression):
(a) Retinal vascular disorders (including but not limited to diabetic retinopathy, retinal vein occlusions and retinopathy of prematurity). The pathophysiology of retinal vascular disorders involves leakage and/or neovascularization from existing retinal vessels.
(b) Subretinal neovascular disorders (including but not limited to neovascular age-related macular degeneration, ocular histoplasmosis, pathologic myopia). The pathophysiology of subretinal neovascular disorders involves the growth of new vessels into the normally avascular outer retina and subretinal space.
(c) intraocular tumors (including ocular melanoma, ocular lymphoma and retinoblastoma),

The driving force for upregularing ocular VEGF levels has been primarily attributed to retinal ischemia. Anti-VHGF antibodies and artificial VEGF trapping proteins are currently being used to treat VEGF-associated eye diseases (such as those listed above) with some promising results. However, these agents are usually used only late in the disease process and do not work for all patients, In addition, since VEGF is important for maintenance of the choriocapillaris, long term anti-VEGF treatment may lead to geographic atrophy of the retina; "Global" ablation of VEGF may lead to adverse effects after long term treatment

### SUMMARY OF THE INVENTION

The present invention is a method for treating retinal disorders through the administration of an effective amount of human chorionic gonadotropin (hCG) antagonist, luteinizing hormone (LH) antagonist or hCG/LH receptor antagonists, optionally in combination with at least one additional bioactive agent. This treatment blocks hormonally-induced VEGF synthesis by blocking LH/hCG receptor activation (either directly or by competitive inhibition) or by blocking the hormones themselves (i.e. by antibodies directed against hormones). This prevents VEGF-induced neovascularization or angiogenesis of the retina (that typically occur as proliferative eye disorders progress).

### BRIEF DESCRIPTION OF THE DRAWINGS

FIG, 1 shows the amino acid sequence of hCG and also shows the amino acids which are glycosylated (either N- or O-linked);
FIG. 2 shows the preretinal nuclei count in a neovascularization experiment conduct using hCG at doses of 1320 and 1600 IU/kg; and
FIG. 3 shows the ocular upregulation of VEGF in hCG administrated mice,

### DESCRIPTION OF PREFERRED EMBODIMENTS

### Terminology

For the purposes of promoting an understanding of the principles of the invention, reference will be made to preferred embodiments and specific language will be used to describe the same. It will nevertheless be understood that no limitation of the scope of the invention is thereby intended, such alteration and further modifications of the invention and such further applications of the principles of the invention as illustrated herein, being contemplated as would normally occur to one skilled in the art to which the invention related.

The following terms shall be used to describe the preferred embodiments present invention. In the absence of a specific definition set forth herein, the terms used to describe the present invention shall be given their common meaning as understood by those of ordinary skill in the art.

Human chorionic gonadotropin (hCG) and luteinizing hormone (LH) share the same receptor. The term "hCG" will refer to any isoform of naturally-occurring or synthetic "human chorionic gonadotropin" and the term "LH" will refer to any isoform of naturally occurring or synthetic "luteinizing hormone." In this patent, when we refer to hCG/LH antagonists, we define hCG/LH antagonist as an "hCG antagonist," "LH antagonist," "hCG/LH antagonist," "hCG receptor antagonist," "LH receptor antagonist," or "hCG/LH receptor antagonist." These terms are used interchangeably, and are intended to include appropriate effective derivatives or fragments of the named compounds, including for example physiologically acceptable esters or salts, employing physiologically acceptable cations or anions.

The term "hGG/LH antagonist" as used herein refers to a disabling moiety which binds either to the hCG or LH hormone, or to the combined receptor thereof

The term "active ingredient" refers to the hCG/LH antagonist.

As used herein, the term "pharmaceutically acceptable carrier," "diluent," "additive" or "excipient" means a chemical composition with which the active ingredient either alone or in combination, can be used to administer the appropriate compound(s) to a patient or subject in therapeutic methods according to the preferred embodiments of the present invention.

As used herein, the term "physiologically acceptable" ester or salt means an ester or salt form of the active ingredient which is compatible with any other ingredients of the pharmaceutical composition, which is not deleterious to the subject to which the composition is to be administered.

As used herein, an "oily" liquid is one which comprises a carbon-containing liquid molecule and which exhibits a less polar character than water.

The term "DR" will stand for "diabetic retinopathy."

The term "patient" or "subject" is used throughout the specification to describe an animal, generally a human, in need, in which hCG/LH antagonist is administered to effect an intended result in that patient or subject. The age of the patient may range from neonate (as would be the case with retinopathy of prematurity) through adult (as would most often be the case with diabetic retinopathy or age-related macular degeneration). The patient may be non-pregnant or pregnant.

The term "effective amount" is used throughout the specification to describe an amount of the present composition. (hCG/LH antagonist) which is used to effect an intended result when used in the methods of the preferred embodiments. In numerous aspects of the present invention the term effective amount is used in conjunction with the treatment of a patient

As used herein, "pharmaceutically acceptable additives" include, but are not limited to, one or more of the following surface active agents; dispersing agents; inert diluents; granulating and disintegrating agents: binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; antifungal agents; stabilizing agents; and pharmaceutically acceptable polymeric or hydrophobic materials. Other "additives" which may be included in the pharmaceutical compositions of the invention are known in the art and described, for example in Genaro, ed. (1985, Remington's Pharmaceutical Sciences, Mack Publishing Co., Easton, PA).

### The Methods in General

The methods of the preferred embodiments may be used to treat a patient suffering from any ocular disorders, including retinal conditions in which hCG or LH may play a role by its promotion of angiogenesis or neovascularization.

The method of treatment involves the administration of an effective amount of ocular human chorionic gonadotropin (hCG) aniagonist, luteinizing hormone (LH) antagonist or hCG/LH receptor antagonists, optionally in combination with at least one additional bioactive agent. This treatment blocks hormonally-induced VEGF synthesis by blocking retinal LH/hCG receptor activation (either directly or by competitive inhibition) or by blocking the hormones themselves (such as by antibodies directed against hormones). This prevents VEGF-induced neovascularization or angiogenesis of the retina (that typically occur as proliferative eye disorders progress). Routes of administration for the treatment may include but are not limited to topical (such as in the form of an eye drop), intraocular, subconjunctival, intravitreal, any form of intraocular, intrathecal, intravenous, parenteral, intraperitoneal, oral, intranasal. Topical application in the form of eye drops is preferred.

### Exemplary Disorders Treated

Examples of angiogenic and/or neovascular ocular disorders in which hCG or LH may play a role include:
(a) Retinal vascular disorders (including but not limited to diabetic retinopathy, retinal vein occlusions and retinopathy of prematurity).
(b) Subretinal vascular disorders (including but not limited to vascular age-related macular degeneration, ocular histoplasmosis, pathologic myopia).
(c) ocular tumors (including ocular melanoma, ocular lymphoma and retinoblastoma).

This method of blocking hormonally induced ocular VEGF synthesis can be used to prevent the progression of ocular tumors. Until now, hCG antagonists have been suggested for use to treat hCG-associated tumors. hCG-associated tumors are tumors that specifically secrete hCG (such as germ cell tumors, certain lung carcinomas and bladder carcinomas). However, the method of this invention is to use ocular hCG/LH antagonists or hCG/LH receptor antagonists to block the progression of any ocular tumor (whether or not the tumor itself secretes hCG) by blocking ocular VEGF synthesis. Increased VEGF levels in the eye (even if they are not made by the tumor itself) may contribute to tumor growth. Though LH/hCG antagonists have been previously proposed for use in ovarian hyperstimulation syndrome, gestational trophoblastic disease, and in hCG-secreting tumors, their use in ocular disorders have never been previously proposed.

### Theoretical Discussion

Although not wishing to be bound by theory, the method of the preferred embodiments appears to involve the regulation of LH/hCG receptor activity in the retina to provide a novel approach to preventing progression of eye disorders involving neovascularization/angiogenesis (in which VEGF may play a role). The method is believed to involve targeting a hormonal signaling pathway that occurs upstream of VEGF production as a new therapeutic target for VEGF-associated ocular diseases Specifically, the preferred embodiment methods appear to focus upon the LH/hCG receptor which is activated by either luteinizing hormone (LH) or by human chorionic gonadotropin (hCG).

### Exemplary hCG/LH Antagonists

Exemplary hCG/LH antagonists include both small molecule antagonists and macromolecule antagonists [molecular weight (MW)> 150 kDa], such as antibody antagonists, Small molecule antagonists are preferred. Small molecule antagonists of less than 150 kDa lend themselves to formulation as an eye drop to provide a convenient and safe mode of delivery. The small molecule antagonist should be small enough to achieve efficient intraocular penetration via eye drop (topical) application, but should be sufficiently large to result in slow clearance from the vitreous. Preferably, they should have a molecular weight of 20 to 80 kDa, more preferably 30-50 kDa.

An example of a small molecule hCG/LH antagonist that may be used in the preferred embodiments, the hCG/LH antagonist may comprise a deglycosylated hCG, a mutant hCG or an hCG molecule which is both deglycosylated and mutant. An example of one class of hCG/LH antagonists is "deglycosylated hCG" and used herein refers to hCG which has a reduced number of carbohydrate residues relative to naturally occurring hCG. In one embodiment, the deglycosylated hCG comprises less N-linked carbohydrates than naturally occurring hCG.

The prior art describes ways to deglycosylate hCG. For example, hCG can be by deglycosylated by neuraminidase treatment or by hydrogen fluoride treatment. Patent Application US2008/0039372 A1 describes embodiments of deglycosylated hCG which comprises a reduced number of N-linked carbohydrates relative to naturally occurring hCG. In one embodiment, the deglycosylated hCG has its alpha and beta subunits ligated together (fused or yoked) and has had 3 of the 4 N-linked carbohydrate sites altered by site specific mutagenesis. hCG/LH antagonists as described in US2008/0039372 represent preferred embodiments of antagonists which are used in the present, application. Alpha or beta subunits of hCG which have been deglycosylated may also be used as inhibitors of hCG for purposes of the present invention. Mutant forms of hCG or the alpha or beta subunits of hCG represent additional hCG/LH antagonists for use in the present invention.

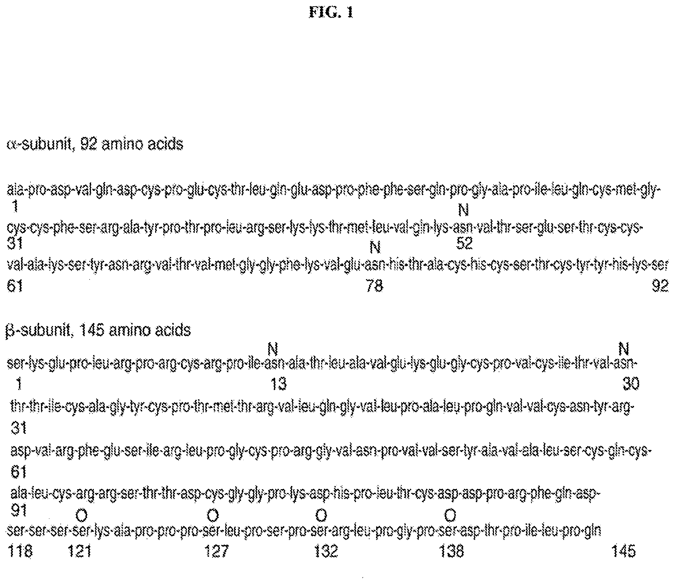

The deglycosylated hCG may comprise a reduced number of sialic acid residues, N-acetyl glucosamine residues, N-galactosamine residues, galactose residues, mannose residues, or fucose residues compared to naturally occurring hCG. The deglycosylated hCG may have at least about 50% to 90% fewer carbohydrates than naturally occurring hCG. In particular, the deglycosylated hCG may have at least about 55%, 60%, 65%, 70%, 75%, 80%, 85%, 90%, 95%, 97%, 99% 99% or 99+% less carbohydrates than naturally occurring hCG. FIG. 1 shows the amino acid sequence of hCG and also shows the amino acids which are glycosylated (either N- or O-linked). These amino acids are asparagine at amino acids 52 and 78 of the alpha subunit of hCG and asparagine at amino acids 13 and 30 and serine at amino acids 121, 127, 132 and 138 of the beta subunit of hCG. Pursuant to the present invention, the glycosyl residues may be modified (generally, shortened or deglycosylated as otherwise described herein) to provide inhibitors of hCG which are useful in the present invention. In addition, mutant hCG molecules which are obtained by modifying one or more amino acid residues within the sequence of hCG may also be used in the present invention. Preferred hCG mutants as antagonists for use in the present invention include mutant hCG molecules which have eliminated glycosylation by substituting an amino acid which cannot be glycosylated for one of the amino acids. The substitution or elimination of one or more amino acids selected from asparagine at amino acids 52 and 78 of the alpha subunit of hCG and asparagine at amino acids 13 and 30 and serine at amino acids 121, 127, 132 and 138 of the beta subunit of hCG represent hCG mutants which may be used as hCG/LH antagonists pursuant to the present invention.

Large molecule hCG/LH antagonists that could be used are antibodies (either monoclonal or polyclonal) or antibody fragments that are directed against LH, against hCG and/or against the LH/hCG receptor. The methodology, technology and skills required for production of such antibodies are readily available. LH antibodies, hCG antibodies and LH/hCG receptor antibodies are commercially available.

### Exemplary Pharmaceutical Compositions

The pharmaceutical compositions to be administered in the preferred embodiments include hCG/LH antagonists in an effective amount either alone, in a form suitable for administration, to a subject, or the pharmaceutical composition may comprise the active ingredient and one or more pharmaceutically acceptable carriers, diluents, additives, excipients, and/or one or more additional ingredients, or some combination of these. Pharmaceutical compositions according to the preferred embodiments may be formulated and administered to deliver a dose of between 1 ng/kg/day and 100 mg/kg/day of hCG/LH antagonist or variant thereof to the patient or subject in need of therapy as otherwise described herein.

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multi-dose unit.

A pharmaceutical composition of the invention may be prepared, packaged, or sold in bulk, as a single unit dose, or as a plurality of single unit doses. As used herein, a "unit dose" is a discrete amount of the pharmaceutical composition comprising a predetermined amount of the active ingredient. The amount of the active ingredient is generally equal to the dosage of the active ingredient which would he administered to a subject or a convenient traction of such a dosage such as, for example, one-half or one-third of such a dosage.

The relative amounts of the active ingredient, the pharmaceutically acceptable carrier, and any additional ingredients in a pharmaceutical composition of the invention will vary, depending upon the identity, size, and condition of the subject treated and further depending upon the route by which the composition is to be administered, By way of example, the composition may comprise less than 0.1% to 100% (w/w) active ingredient. Controlled- or sustained-release formulations of a pharmaceutical composition of the invention may be made using conventional technology.

Pharmaceutical compositions that are useful in the methods of the preferred embodiments may be administered, prepared, packaged, and/or sold in formulations suitable for oral, rectal, vaginal, parenteral, topical, pulmonary, intranasal, buccal, ophthalmic (including without limitation topical, intraocular, intravitreal, subconjunctival), suppository, aerosol, or another route of administration. The route(s) of administration will be readily apparent to the skilled artisan and will depend upon any number of factors including the type and severity of the disease being treated, the type and age of patient being treated, and the like.

Topical application formulations for use as eye drops are preferred. Such formulations may, for example, be in the form of eye drops including, for example, a 0,1-1.0% (W/W) solution or suspension of the active ingredient in an aqueous or oily liquid carrier. Such drops may further comprise buffering agents, salts, or one or more other of the additional ingredients described herein.

Other possible formulations, such as nanoparticles, liposomes, reseated erythrocytes, and immunologically based systems may also be used to administer, for example, peptides, fragments, or derivatives, and/or a nucleic acid encoding the same according to the methods of the invention.

A formulation of a pharmaceutical composition of the invention suitable for oral administration may be prepared, packaged, or sold in the form of a discrete solid dose unit including, but not limited to, a tablet, a hard or soft capsule, a cachet, a troche, or a lozenge, each containing a predetermined amount of the active ingredient. Other formulations suitable for oral administration include, but are not limited to, a powdered or granular formulation, an aqueous or oily suspension, an aqueous or oily solution, or an emulsion.

Liquid formulations of a pharmaceutical composition of the invention which are suitable for oral administration may be prepared, packaged, and sold either in liquid form or in the form of a dry product intended for reconstitution with water or another suitable vehicle prior to use.

The compound can be administered to a subject as frequently as several times daily, or it may be administered less frequently, such as once a day, once a week, once every two weeks, once a month, or even less frequently, such as once every several months or even once a year or less. The frequency of the dose will be readily apparent to the skilled artisan and will depend upon any number of factors, such as, but not limited to, the type and severity of the retinal disease being treated, route of administration, the age of the subject.

Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the invention is contemplated include, but are not limited to, humans and other primates, mammals including commercially relevant mammals such as cattle, pigs, horses, sheep, cats, and dogs.

It will be recognized by one of skill in the art that the various embodiments of the invention as described above relating to specific methods of treating ocular conditions or ameliorating the disease state and/or condition or its symptoms may relate within context to the treatment or resolution of symptomatology not specifically mentioned herein, or may involve different circumstances of symptomatology.

### Discussion of Examples

Example 1 below demonstrates that the administration of hCG promotes abnormal retinal neovascularization in the mouse model of oxidative-induced retinopathy effect, of hCG on retinal neovascularization in a mouse model of oxygen-induced retinopathy (OIR). The OIR mouse model has been the cornerstone of many studies investigating proliferative retinopathies (such as diabetic retinopathy and retinopathy of prematurity). The data from this experiment supports the concept that LH/hCG signaling can influence retinal neovascularization. In example 1 below, hCG was used because it has a much longer half-life (>24 hours) than LH (20 minutes) and thus, is more practical for use in experimentation involving the activation of the LH/hCG receptor. The use of hCG as an LH surrogate is common in both clinical and pre-clinical investigations involving LH.

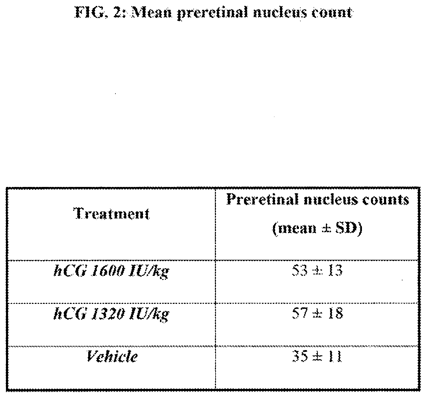

### Example 1: See FIG. 2

The following experiment was conducted to demonstrate that hCG at doses of 1320 and 1600 IU/kg significantly increases retinal neovascularization in the mouse model of oxidative-induced retinopathy. Thus, hCG/LH antagonist may help treat ocular disorders involving neovascularization.

### Objective

The aim of this study was to assess the effect of human chorionic gonadotropin hormone (hCG) delivered intraperitoneally on retinal neovascularization in the mouse model of oxygen-induce retinopathy (OIR).

### Methods

Three (3) litters of five (5) pup mice were used, one in each treatment group. The retinopathy was induced in Day-7 postnatal animals (P7) by a 5-day exposure to a 75% oxygen atmosphere (from P7 to P12). Animals were then kept in normoxia. Animals were treated by once daily intraperitoneal (ip) administrations of hCG at 1320 or 1600 IU/kg and vehicle control for 10 consecutive days (from P5 to P14). On P17, both eyeballs were removed and processed for histology.

The retinal neovascularization was evaluated by counting preretinal nuclei, i.e. the nuclei from the new vessels extending into the vitreal side of the internal limiting membrane in histological sections from both eyes.

### Results: See FIG. 2

Animal behavior and body weight was not altered by hCG treatments, regardless of the dose, in comparison with the vehicle treated litter. Two pup mice from the vehicle group were found dead on P6 and P7. These deaths were due to the mother abandoning of the pups. hCG hormone daily administered intraperitoneally at dosed of 1320 and 1600 IU/kg significantly increased by 63% (p = 0.004) and 51% (p = 0.0085) preretinal nucleus count, respectively.

In these experimental conditions, multiple intraperitoneal administrations of human chorionic gonadotropin (hCG) were well tolerated by the pup mice. hCG at doses of 1320 and 1600 IU/kg significantly increased retinal neovascularization in the mouse model of OlR. Example 2:

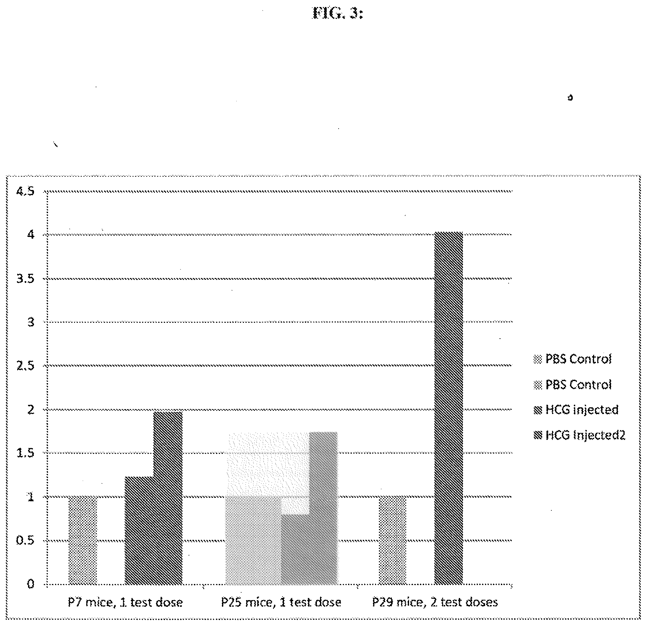

HCG may promote ocular angiogenesis by functioning as an ocular VEGF regulator. VEGF has been implicated in diabetic retinopathy, macular degeneration and retinopathy of prematurity. Thus, hCG/LH antagonist may be helpful in these disorder. See FIG. 3.

In the uterine vasculature, the pro-angiogenic hormone, hCG, is an important regulator of vascular endothelial growth factor (VEGF) and two VEGF receptors, (VEGFR-1, VEGFR-2) I propose that hCG also functions as an ocular VEGF regulator. The hallmark of early stage ROP, regression of immature retinal vasculature, results from hyperoxia-induced suppression of VEGF. The abnormal neovascularization characteristic of advanced stage ROP results from the retina's exuberant *upregulation* of VEGF to overcompensate for the earlier oxygen-induced VEGF suppression. The following experiment(s) was performed.
Relative Quantity Chart (one control from each group set as calibrator)
Murine Neural Retinal VEGFA expression post intraperitoneal administration of PBS (control vehicle) versus hCG (test compound)
P=postnatal day (i.e. P7=postnatal day 7);
(SDS v1.4, ddCT study, Applied Biosystems 7500 Fast Real-Time PCR System).
1320 IU/kg intraperitoneal human chorionic gonadotropin (hCG) or phosphate buffered saline were intraperitioneally administered to G57BL/6 wild type mice.
(a) Group 1: P7 murine neonates, were injected with either hCG or PBS with retinal processing one day later. (Of the 4 mice that had been born in the litter, one was a runt. Since runts are typically behind in ocular development, it was not used as a control. Therefore Group I has one control (PBS) and two test mice (hCG). Each of the two test mice showed increase in retinal VEGFa expression, though the increase reached significance (p<0.05) in one of the two. Averaging the two test mice results, there was 70% increase in retinal VEGFa expression in immature HCG treated retinae compared to control.
(b) Group 2: P25 young adult mice, received either an injection of hCG or PBS with retinal processing the next day. There were two controls and two test mice in this group. Though one of the two test mice had a nonsignificant 20% decline, in retinal VEGFa expression, the other test mouse exhibited a 70% significant (p<0.05) increase in VEGFa expression over each control.
(c) Group 3: P29/P30 young adult mice, received 2 doses of either hCG or PBS, one dose on each of two consecutive days with retinal processing the day after the second dose. There was one control (PBS) and one test mouse (hCG) in this group. There was a 400% significant (p<0.05) increase in VEGFa expression in the test mouse vs the control.

The results from the above experiments are shown in attached FIG. 3. They provide evidence that hCG exhibited significant upregulation of VEGF in the mice which were tested.

## Claims

1. A hCG/LH antagonist for use in a method for reducing the likelihood or inhibiting the progression of an ocular disorder in a patient in need, the use comprising administering to said patient an effective amount of the hCG/LH antagonist, optionally in combination with an additional bioactive agent effective for ameliorating the effects of said ocular disorder.

2. The hCG/LH antagonist for use of claim 1 in which said hCG/LH antagonist is a small molecule entity having a molecular weight of less than 150 kDa.

3. The hCG/LH antagonist for use of claim 1 in which said hCG/LH antagonist is a small molecule entity having a molecular weight of from 20 to 80 kDa;
in which said hCG/LH antagonist is, optionally, applied topically to the eye.

4. The hCG/LH antagonist for use of claim 1 in which said hCG/LH antagonist is a small molecule entity having a molecular weight of 30-50 kDa;
in which said hCG/LH antagonist is, optionally, applied topically to the eye.

5. The hCG/LH antagonist for use according to any of claims 1-4 wherein said hCG/LH antagonist is a deglycosylated hCG, a mutant hCG or an hCG mutant which has also been deglycosylated.

6. The hCG/LH antagonist for use according to any of claims 1-4 wherein said hCG/LH antagonist is deglycosylated hCG or a mutant hCG which is also deglycosylated, said deglycosylated hCG comprising less N-linked carbohydrates than naturally occurring hCG.

7. The hCG/LH antagonist for use according to any of claims 1-4 wherein said hCG/LH antagonist is deglycosylated hCG or a mutant hCG which is also deglycosylated, said deglycosylated hCG having at least 55% less carbohydrates than naturally occurring hCG;
wherein, optionally, said deglycosylated hCG has at least 95% less carbohydrates than naturally occurring hCG.

8. The hCG/LH antagonist for use according to any of claims 1-7 wherein said hCG/LH antagonist is coadministered in combination with an additional bioactive agent;
wherein, optionally, said additional bioactive agent is at least one agent selected from the group consisting of ranibizumab, aflibercept, pegatanib, verteporfin, triamcinolone and bevacizumab.

9. The hCG/LH antagonist for use according to any of claims 1-8 wherein the ocular disorder is a retinal disorder.

10. The hCG/LH antagonist for use according to any of claims 1-8 wherein said ocular disorder is selected from the group consisting of diabetic retinopathy, retinal vein occlusions, and retinopathy of prematurity; or
wherein said ocular disorder is selected from a group of subretinal neovascular disorders selected from the group consisting of macular degeneration, ocular histoplasmosis, and pathologic myopia; or
wherein said ocular disorder is selected from the group consisting of intraocular tumors which are NOT known to secrete hCG or LH.

11. The hCG/LH antagonist for use according to claim 1 wherein said hCG/LH antagonists are hCG/LH antibodies.

12. A pharmaceutical composition comprising an effective amount of an hCG/LH antagonist in combination with at least one agent selected from the group consisting of ranibizumab, aflibercept, pegatanib, verteporfin, triamcinolone and bevacizumab, in combination with a carrier, additive and/or excipient.

13. A pharmaceutical composition comprising an ophthalmic solution of a small molecule hCG/LH antagonist having a molecular weight of from 20 to 80 kDa.

14. A pharmaceutical composition comprising a topical application eye drop solution of a small molecule hCG/LH antagonist having a molecular weight of from 20 to 80 kDa.

15. The pharmaceutical composition of claim 13 or 14 in which said small molecule hCG/LH antagonist has a molecular weight of 30-50 kDa.

## Patentansprüche

1. hCG/LH-Antagonist für die Verwendung in einem Verfahren für die Reduzierung der Wahrscheinlichkeit oder Inhibierung des Fortschreitens einer okularen Störung bei einem Patienten, der dies benötigt, wobei die Verwendung die Verabreichung an den Patienten einer wirksamen Menge des hCG/LH-Antagonisten, optional zusammen mit einem zusätzlichen bioaktiven Mittel, das für die Linderung der Effekte der okularen Störung wirksam ist, umfasst.

2. hCG/LH-Antagonist für die Verwendung nach Anspruch 1, in der der hCG/LH-Antagonist eine kleinmolekulare Einheit ist, die ein Molekulargewicht von weniger als 150 kDa aufweist.

3. hCG/LH-Antagonist für die Verwendung nach Anspruch 1, in der der hCG/LH-Antagonist eine kleinmolekulare Einheit ist, die ein Molekulargewicht von 20 bis 80 kDa aufweist;
in der der hCG/LH-Antagonist optional topisch an dem Auge angewendet wird.

4. hCG/LH-Antagonist für die Verwendung nach Anspruch 1, in der der hCG/LH-Antagonist eine kleinmolekulare Einheit ist, die ein Molekulargewicht von 30-50 kDa aufweist;
in der der hCG/LH-Antagonist optional topisch an dem Auge angewendet wird.

5. hCG/LH-Antagonist für die Verwendung nach einem der Ansprüche 1-4, wobei der hCG/LH-Antagonist ein deglycosyliertes hCG, ein mutiertes hCG oder ein hCG-Mutant, der auch deglycosyliert wurde, ist.

6. hCG/LH-Antagonist für die Verwendung nach einem der Ansprüche 1-4, wobei der hCG/LH-Antagonist deglycosyliertes hCG oder ein mutiertes hCG ist, das auch deglycosyliert ist, wobei das deglycosylierte hCG weniger N-verknüpfte Kohlenhydrate umfasst als natürlich vorkommendes hCG.

7. hCG/LH-Antagonist für die Verwendung nach einem der Ansprüche 1-4, wobei der hCG/LH-Antagonist deglycosyliertes hCG oder ein mutiertes hCG ist, das auch deglycosyliert ist, wobei das deglycosylierte hCG mindestens 55 % weniger Kohlenhydrate aufweist als natürlich vorkommendes hCG;
wobei optional das deglycosylierte hCG mindestens 95 % weniger Kohlenhydrate aufweist als natürlich vorkommendes hCG.

8. hCG/LH-Antagonist für die Verwendung nach einem der Ansprüche 1-7, wobei der hCG/LH-Antagonist zusammen mit einem zusätzlichen bioaktiven Mittel gemeinsam verabreicht wird;
wobei optional das zusätzliche bioaktive Mittel mindestens ein Mittel ist, das aus der Gruppe ausgewählt ist, die aus Ranibizumab, Aflibercept, Pegaptanib, Verteporfin,
Triamcinolon und Bevacizumab besteht.

9. hCG/LH-Antagonist für die Verwendung nach einem der Ansprüche 1-8, wobei die okulare Störung eine retinale Störung ist.

10. hCG/LH-Antagonist für die Verwendung nach einem der Ansprüche 1-8, wobei die okulare Störung aus der Gruppe ausgewählt ist, die aus diabetischer Retinopathie, retinalen Venenverschlüssen und Frühgeborenen-Retinopathie besteht; oder
wobei die okulare Störung aus einer Gruppe von subretinalen neovaskulären Störungen ausgewählt ist, die aus der Gruppe ausgewählt sind, die aus Makuladegeneration, okularer Histoplasmose und pathologischer Myopie besteht; oder
wobei die okulare Störung aus der Gruppe ausgewählt ist, die aus intraokularen Tumoren besteht, von denen NICHT bekannt ist, dass sie hCG oder LH absondern.

11. hCG/LH-Antagonist für die Verwendung nach Anspruch 1, wobei die hCG/LH-Antagonisten hCG/LH-Antikörper sind.

12. Pharmazeutische Zusammensetzung, die eine wirksame Menge eines hCG/LH-Antagonisten zusammen mit mindestens einem Mittel, das aus der Gruppe ausgewählt ist, die aus Ranibizumab, Aflibercept, Pegaptanib, Verteporfin, Triamcinolon und Bevacizumab besteht, zusammen mit einem Träger, Additiv und/oder Hilfsstoff umfasst.

13. Pharmazeutische Zusammensetzung, die eine ophthalmische Lösung eines kleinmolekularen hCG/LH-Antagonisten umfasst, der ein Molekulargewicht von 20 bis 80 kDa aufweist.

14. Pharmazeutische Zusammensetzung, die eine Augentropfenlösung zur topischen Anwendung eines kleinmolekularen hCG/LH-Antagonisten umfasst, der ein Molekulargewicht von 20 bis 80 kDa aufweist.

15. Pharmazeutische Zusammensetzung nach Anspruch 13 oder 14, in der der kleinmolekulare hCG/LH-Antagonist ein Molekulargewicht von 30-50 kDa aufweist.

## Revendications

1. Antagoniste de hCG/LH destiné à une utilisation dans une méthode de réduction de la probabilité ou d'inhibition de la progression d'un trouble oculaire chez un patient en ayant besoin, l'utilisation comprenant l'administration audit patient d'une quantité efficace de l'antagoniste de hCG/LH, éventuellement en combinaison avec un agent bioactif supplémentaire efficace pour améliorer les effets dudit trouble oculaire.

2. Antagoniste de hCG/LH destiné à une utilisation selon la revendication 1, où ledit antagoniste de hCG/LH est une petite entité moléculaire ayant un poids moléculaire inférieur à 150 kDa.

3. Antagoniste de hCG/LH destiné à une utilisation selon la revendication 1, où ledit antagoniste de hCG/LH est une petite entité moléculaire ayant un poids moléculaire allant de 20 à 80 kDa ;
où ledit antagoniste de hCG/LH est, éventuellement, appliqué par voie topique à l'oeil.

4. Antagoniste de hCG/LH destiné à une utilisation selon la revendication 1, où ledit antagoniste de hCG/LH est une petite entité moléculaire ayant un poids moléculaire de 30-50 kDa ;
où ledit antagoniste de hCG/LH est, éventuellement, appliqué par voie topique à l'oeil.

5. Antagoniste de hCG/LH destiné à une utilisation selon l'une quelconque des revendications 1-4, où ledit antagoniste de hCG/LH est une hCG déglycosylée, une hCG mutante ou une hCG mutante qui a également été déglycosylée.

6. Antagoniste de hCG/LH destiné à une utilisation selon l'une quelconque des revendications 1-4, où ledit antagoniste de hCG/LH est une hCG déglycosylée ou une hCG mutante qui est également déglycosylée, ladite hCG déglycosylée comprenant moins de glucides N-liés que la hCG présente naturellement.

7. Antagoniste de hCG/LH destiné à une utilisation selon l'une quelconque des revendications 1-4, où ledit antagoniste de hCG/LH est une hCG déglycosylée ou une hCG mutante qui est également déglycosylée, ladite hCG déglycosylée comprenant au moins 55% en moins de glucides que la hCG présente naturellement ;
où, éventuellement, ladite hCG déglycosylée comprend au moins 95% en moins de glucides que la hCG présente naturellement.

8. Antagoniste de hCG/LH destiné à une utilisation selon l'une quelconque des revendications 1-7, où ledit antagoniste de hCG/LH est coadministré en combinaison avec un agent bioactif supplémentaire ;
où, éventuellement, ledit agent bioactif supplémentaire est au moins un agent choisi dans le groupe constitué par le ranibizumab, l'aflibercept, le pégaptanib, la vertéporfine, la triamcinolone et le bévacizumab.

9. Antagoniste de hCG/LH destiné à une utilisation selon l'une quelconque des revendications 1-8, où le trouble oculaire est un trouble rétinien.

10. Antagoniste de hCG/LH destiné à une utilisation selon l'une quelconque des revendications 1-8, où ledit trouble oculaire est choisi dans le groupe constitué par la rétinopathie diabétique, les occlusions veineuses rétiniennes, et la rétinopathie du prématuré ; ou
où ledit trouble oculaire est choisi dans le groupe constitué par les troubles néovasculaires sous-rétiniens choisis dans le groupe constitué par la dégénérescence maculaire, l'histoplasmose oculaire, et la myopie pathologique ; ou
où ledit trouble oculaire est choisi dans le groupe constitué par les tumeurs intra-oculaires qui ne sont PAS connues comme sécrétant la hCG ou la LH.

11. Antagoniste de hCG/LH destiné à une utilisation selon la revendication 1, où lesdits antagonistes de hCG/LH sont des anticorps anti-hCG/LH.

12. Composition pharmaceutique comprenant une quantité efficace d'un antagoniste de hCG/LH en combinaison avec au moins un agent choisi dans le groupe constitué par le ranibizumab, l'aflibercept, le pégaptanib, la vertéporfine, la triamcinolone et le bévacizumab, en combinaison avec un véhicule, un additif et/ou un excipient.

13. Composition pharmaceutique comprenant une solution ophtalmique d'un antagoniste de hCG/LH à petite molécule ayant un poids moléculaire allant de 20 à 80 kDa.

14. Composition pharmaceutique comprenant une solution de gouttes ophtalmiques pour application topique d'un antagoniste de hCG/LH à petite molécule ayant un poids moléculaire allant de 20 à 80 kDa.

15. Composition pharmaceutique selon la revendication 13 ou 14, où ledit antagoniste de hCG/LH à petite molécule possède un poids moléculaire de 30-50 kDa.
